# EUROPEAN PATENT SPECIFICATION

(11) **EP 0 371 728 B1**
(45) Date of publication and mention of the grant of the patent: **08.01.1997**
(21) Application number: 89312289.5
(22) Date of filing: 27.11.1989
(51) Int. Cl.: A01N 59/00, A61K 47/02, A61K 9/06, A61L 2/18

(54) **Aqueous ophthalmic solutions and method for preserving same**
Wässrige Augenlösung und Verfahren zur Konservierung derselben
Solution ophtalmique aqueuse et son procédé de conservation

(30) Priority: 29.11.1988 US 277791
(43) Date of publication of application: 06.06.1990
(73) Proprietor: ALLERGAN, INC, Irvine, California 92715 (US)
(72) Inventor: Dziabo, Anthony J., Jr., El Toro California 92630 (US); Ripley, Paul S., Irvine California 92714 (US)
(74) Representative: Hutchins, Michael Richard

(56) References cited:
- US-A- 3 123 521
- US-A- 4 499 077
- US-A- 4 689 215

## Description

### Background of the Invention

### 1. Field of the Invention

The present invention relates to preserving ophthalmic solutions. More particularly it relates to the use of stabilized chlorine dioxide to preserve ophthalmic solutions.

### 2. Brief Description of the Prior Art

The use of contact lenses has become widespread as a replacement for conventional eye glasses because of the improved vision obtained by the wearer or for aesthetic reasons. Contact lenses accumulate microorganisms and cellular debris from the eye. Thus, the lenses must be periodically removed and cleaned to prevent irritation of the eye or infection. Solutions used in lens care must be preserved by some means to interdict introducing microbial contaminants onto contact lenses or the eye. Disinfecting preparations are part of the regimen indicated for contact lens care.

Numerous ophthalmic solutions have heretofore been used with lenses. The composition of the ophthalmic solution will often be dictated by the polymeric materials employed in the fabrication of the contact lens. Because of the chemical composition of most ophthalmic solutions, the contact lenses cleaned and soaked in such solutions must be rinsed prior to placement in the wearer's eye to prevent irritation of the eye.

US Patent No. 4,499,077 discloses antimicrobial solutions containing a combination of a quaternary ammonium compound and stabilised chlorine dioxide for treating contact lenses. This document states explicitly that it is not possible to obtain ophthalmically acceptable effective antimicrobial compositions containing chlorine dioxide alone.

U.S. Patent Nos. 4,696,811 and 4,689,215 disclose the use of stabilized chlorine dioxide for the treatment and prevention of oral disease, the reduction of malodor, as an anti-plaque agent, an anti-gingivitis and anti-periodontitis agent, as well as a denture soak and a contact lens soak. That is, the two before-referenced patents disclose the use of 0.005 percent to 0.02 percent stabilized chlorine dioxide in sterilized water as a contact lens soaking formulation. However, the references are void of any teaching or suggestion that stabilized chlorine dioxide can be incorporated into an aqueous saline ophthalmic solution as a preservative for such a solution.

Thus, while the prior art compositions (including the composition disclosed in U.S. Patent Nos. 4,696,811 and 4,689,215) have found some acceptance, such prior art compositions generally have limited efficiency in the cleaning and soaking of contact lenses, or such compositions are restricted to contact lenses fabricated of specific polymeric formulations.

Problems have also been encountered in the use of the prior art ophthalmic solutions for the cleansing and soaking of contact lenses in that such solutions often become contaminated or deteriorate when exposed to the atmosphere once the seal of the container containing such solution has been broken. Microorganisms or other impurities often contaminate the solution which requires the solution to be discarded. Thus, there exists a need for an aqueous ophthalmic solution having an extended life; that is, the incorporation into such ophthalmic solution of a constituent which functions as a preservative or disinfectant. It is to such a solution and method for preserving an aqueous saline ophthalmic solution that the present invention is directed.

### Summary of the Invention

Broadly, the present invention relates to an aqueous ophthalmic solution containing an effective minor amount of stabilized chlorine dioxide to effectively preserve the ophthalmic solution.

According to the present invention there is provided a preserved ophthalmic formulation comprising an ophthalmically acceptable aqueous medium and, included therein, stabilised chlorine dioxide in an amount in a range from 0.0002 to 0.02 weight/volume % effective to act as the sole preservative in said formulation, an ophthalmically acceptable buffer component in an amount effective to maintain said formulation at a pH in a range from 6.8 to 8 and an ophthalmically acceptable inorganic salt in an amount effective to maintain the tonicity of said formulation at an ophthalmically acceptable level.

The preserved ophthalmic formulations of the invention preferably consist essentially of the ophthalmically acceptable aqueous medium, stabilised chlorine dioxide, ophthalmically acceptable buffer component and said ophthalmically acceptable inorganic salt.

Particular and preferred embodiments of the invention are as set forth in the claims appended hereto.

In one aspect the present invention relates to an aqueous ophthalmic solution comprising purified water as a vehicle, from about 0.0002 to about 0.02 weight/volume percent stabilized chlorine dioxide as the sole preservative, and an effective minor amount of an ophthalmically acceptable inorganic salt to provide the ophthalmic solution with a tonicity value substantially corresponding to the tonicity value of fluids of an eye. To stabilize the pH of the ophthalmic solution, the ophthalmic solution also includes an effective minor amount of a buffering agent. To provide the ophthalmic solution with a pH substantially corresponding to the pH of the fluids of the eye, and to eliminate the rinsing of the contact lenses prior to the insertion of the contact lens into the eye, the pH of the ophthalmic solution can be adjusted, if required, by addition of an acid or a base so that the ophthalmic solution has an acceptable physiological pH (i.e., a pH in the range of from about 6.8 to about 8).

An object of the present invention is to provide a preservative for ophthalmic solutions.

Another object of the present invention, while achieving the before-stated object, is to provide an ophthalmic solution wherein the solution has a pH and tonicity value substantially corresponding to such values of the fluids of the human eye.

Another object of the present invention, while achieving the before-stated objects, is to provide an aqueous saline ophthalmic solution having incorporated therein a preserving agent such that the integrity of the saline solution is maintained.

Yet another object of the present invention, while achieving the before-stated objects, is to provide an improved composition useful as a disinfectant for ophthalmic devices.

Other objects, advantages and features of the present invention will become apparent from the following detailed description when read in conjunction with the appended claims.

### Detailed Description

The incorporation of a preserving amount of stabilized chlorine dioxide into an ophthalmic formulation has been found to be an effective preservative for ophthalmic formulations. The preserving amount of stabilized chlorine dioxide incorporated into an ophthalmic formulation (that is, to prevent microbial growth in the formulation), can vary widely but will generally be an amount sufficient to reserve the integrity of the formulation.

When incorporating a preserving amount of stabilized chlorine dioxide into an aqueous ophthalmic solution one can maintain the lenses in contact with the solution without any substantial degradation of the lenses. The cleansed and soaked lenses can be placed directly into the wearer's eye without the additional requirement of rinsing to remove residual solution therefrom. Thus, contamination of the clean lenses is substantially eliminated prior to placement in the wearer's eye.

The term "stabilized chlorine dioxide" is well known in the industry and by those skilled in the art. U.S. Patent No. 2,271,242 discloses a form of stabilized chlorine dioxide and a method for producing same which can be used as a preservative for aqueous ophthalmic solutions. A commercially available stabilized chlorine dioxide which can be utilized in the practice of the present invention is the proprietary stabilized chlorine dioxide of Bio-Cide International, Inc. of Norman, Oklahoma.

The term "aqueous ophthalmic solution" as used herein is to be understood to mean a solution containing sterilized water as the vehicle and having at least one other component, such as an ophthalmically acceptable inorganic salt, which can be administered to or placed in the eye; and wherein the solution will not possess toxic properties or have a deleterious effect on the tissue of the eye. That is, such solutions will not cause stinging or discomfort, redness or other adverse reactions to the eye under normal use conditions.

The term "ophthalmically acceptable inorganic salt" as used herein is to be understood to mean any inorganic salt which is capable of providing the ophthalmic solution with the desired tonicity values and which does not irritate or cause damage to the tissue of the eye.

As previously stated, one aspect of the present invention resides in the use of a preserving amount of stabilized chlorine dioxide in aqueous ophthalmic formulations, particularly a saline ophthalmic solution; or as an ingredient in the formulation of an aqueous ophthalmic solution, particularly a saline solution. In each instance it has been found that ophthalmic devices contacted with an ophthalmic solution containing a preserving amount of stabilized chlorine dioxide do not have to be rinsed to remove residual solution prior to use. Similarly, when such solutions are employed in the regimen of contact lenses, the contact lenses can be placed in a wearer's eye, without rinsing, without irritation or adverse effects occurring to the tissue of the eye, and without discomfort.

The amount of stabilized chlorine dioxide incorporated in the ophthalmic formulation as a preservative can vary widely provided that such amount effectively prevents microbial growth in the formulation. Generally, microbial growth in the ophthalmic formulation can be prevented when the amount of stabilized chlorine dioxide introduced into the formulation is from about 0.0002 to about 0.02 weight/volume percent of the solution, desirably from about 0.004 to about 0.01 weight/volume percent.

In order to insure that the aqueous ophthalmic solution containing a preserving amount of stabilized chlorine dioxide does not irritate one's eye, it is desirable that the ophthalmic solution have a pH value of from about 6.8 to about 8 so that the pH of the ophthalmic solution substantially corresponds to the pH value of the fluids in the eye, or which can be tolerated by the eye without causing any discomfort or irritation.

To stabilize the ophthalmic solution at the desired pH, an effective minor amount of a buffering agent is incorporated into the ophthalmic solution. The effective minor amount of buffering agent employed to buffer the ophthalmic solution at a pH of from about 6.8 to about 8 can vary widely and will depend to a large degree on the particular buffering agent employed, as well as the chemical composition of the ophthalmic solution. However, desirable results have been obtained when the amount of buffering agent incorporated into the aqueous ophthalmic solution to stabilize the solution at the acceptable physiological pH is from about 0.05 to about 1 weight/volume percent of the buffering agent.

Any suitable buffering agent can be employed which is compatible with the other ingredients of the ophthalmic solution, and which does not have deleterious or toxic properties which could harm the eye. Examples of suitable buffering agents are boric acid, sodium borate, sodium phosphates (including mono, di-and tribasic phosphates, such as sodium phosphate monobasic monohydrate, sodium phosphate dibasic heptahydrate, and mixtures thereof). It should be noted that any other suitable buffering agent can be employed to stabilize the pH of the ophthalmic solution so that the ophthalmic solution is provided with an acceptable physiological pH, and the before-mentioned buffering agents are merely examples of such buffering agents. Further, since buffering agents are well known in the art no further examples of such buffering agents which can be utilized in the ophthalmic solutions of the present invention are believed necessary.

When it is determined that the buffered ophthalmic solution does not have a pH value of from about 6.8 to about 8, the pH of the aqueous buffered ophthalmic solution can be adjusted by the addition of an effective amount of either a base or an acid, as the case may be. Any suitable base or acid can be employed to adjust the pH of the aqueous buffered ophthalmic solution which does not provide the ophthalmic solution with toxic or deleterious properties which could harm either ophthalmic devices or the eye. An example of a base which can be used to adjust the pH of the aqueous buffered ophthalmic solution is 1 N sodium hydroxide; and an example of an acid which can be used to adjust the pH of the aqueous buffered ophthalmic solution is 1 N hydrochloric acid.

As set forth above, the integrity of an ophthalmic solution can be enhanced by the incorporation of from about 0.0002 to about 0.02 weight/volume percent stabilized chlorine dioxide. That is, the presence of stabilized chlorine dioxide in an ophthalmic solution greatly enhances the useful or shelf life of the ophthalmic solution.

When formulating an aqueous ophthalmic solution in accordance with the present invention, stabilized chlorine dioxide and an ophthalmically acceptable inorganic salt or other suitable tonicity imparting agent are admixed with sterile water to provide an ophthalmic solution having a tonicity value substantially corresponding to the tonicity value of fluids of the eye. The amount of water employed as the vehicle in the ophthalmic solution will vary depending upon the amount of the stabilized chlorine dioxide and the ophthalmically acceptable inorganic salt and/or other suitable tonicity imparting agents employed in the formulation.

The amount of ophthalmically acceptable inorganic salt utilized can vary widely provided that the amount of the inorganic salt employed is sufficient to provide the ophthalmic solution with the desired tonicity value. Generally the ophthalmic solution will have the desired tonicity value when the amount of ophthalmically acceptable inorganic salt employed in the formulation of the ophthalmic solution is from about 0.5 to about 0.9 weight/volume percent.

Typical of such ophthalmically acceptable inorganic salts are alkali metal chlorides and alkaline earth metal chlorides, such as sodium chloride, potassium chloride, calcium chloride and magnesium chloride. Because it is desirable that one not have to remove residual aqueous ophthalmic solution from the contact lenses after the soaking and cleansing procedure prior to use, the pH of the ophthalmic solution should substantially correspond with the pH of the fluids of the eye. When it is determined that the pH of the ophthalmic solution is not within an acceptable physiological pH, (i.e., a pH in the range of from about 6.8 to about 8), the pH of the ophthalmic solution can be adjusted by the addition of a base or acid, such as 1 N hydrochloric acid or 1 N sodium hydroxide, so that the solution has an acceptable physiological pH.

In order to more fully describe the present invention the following examples are set forth. However, the examples are merely illustrative in purpose and are not intended to be limiting upon the inventive concept as set forth in the appended claims.

### EXAMPLE I

A series of experiments were performed to determine the antimicrobial properties of a borate buffered saline solution preserved with stabilized chlorine dioxide. The stabilized chlorine dioxide employed was the proprietary stabilized chlorine dioxide of Bio-Cide International, Inc. of Norman, Oklahoma. The concentration of the stabilized chlorine dioxide added to the borate buffered saline solution was varied.

The borate buffered saline solution had the following composition:

| Ingredients | Percent (Weight/Volume) |
|---|---|
| Sodium Chloride USP | 0.85 |
| Boric Acid NF | 0.10 |
| Purified Water USP* | To 100 ml |

| | |
|---|---|
| *Quantity sufficient (Q.S.) to provide 100 ml of solution. | |

The pH of the buffered solution was adjusted by the addition of either hydrochloric acid NF or sodium hydroxide NF so that the pH of the saline solution was within the range of from about 7.7 to 7.9.

The stabilized chlorine dioxide was added to the borate buffered saline solution in the following concentrations:

| Percent (Weight/Volume) |
|---|
| 0.005 |
| 0.004 |
| 0.003 |
| 0.002 |

Each of the above concentrations of stabilized chlorine dioxide exhibited the desired preservative properties for the borate buffered saline solution. Further, all four concentrations of the stabilized chlorine dioxide exhibited good antimicrobial activity, with the three highest concentrations achieving total bacterial kill after 24 hours. Tests indicated that total kill of bacteria was achieved by the solution containing 0.002 weight/volume percent stabilized chlorine dioxide after seven days.

### EXAMPLE II

To compare the preservative efficacy of stabilized chlorine dioxide on a borate buffered ophthalmic solution, a preserving amount of stabilized chlorine dioxide having a raw material age of 54 months was utilized in one sample; and a similar preserving amount of stabilized chlorine dioxide having a raw material age of 2 months was utilized in a second sample. Each of the samples of stabilized chlorine dioxide was the proprietary stabilized chlorine dioxide of Bio-Cide International, Inc. of Norman, Oklahoma, under the trademark Purogene. No aging effect was detected between the two samples and their use as a preservative for borate buffered saline solutions. However, the aged stabilized chlorine dioxide (54 month age) possessed a slightly superior activity against the yeast C. albicans.

### EXAMPLE III

A preservative efficacy test was performed on a borate buffered saline solution having a composition similar to that of Example I wherein 0.005 weight/volume percent stabilized chlorine dioxide was added to the borate buffered solution and the resulting mixture stored for 90 days at 45 degrees Centigrade. At the end of the storage period the sample was examined and it was determined that the stabilized chlorine dioxide was an effective preservative for a borate buffered saline solution.

### EXAMPLE IV

An experiment was conducted to determine if a borate buffered saline solution containing 0.005 weight/volume percent stabilized chlorine dioxide met the USP efficacy criteria for ophthalmics as set forth in the U.S. Pharmacopeia (USP XXI, 1985). The stabilized chlorine dioxide employed was the proprietary stabilized chlorine dioxide of Bio-Cide International, Inc. of Norman, Oklahoma. The criteria for preservatives requires that a 99.9% reduction of microbes challenge occur within 14 days of contact with the product being tested; and that no growth of yeast and fungi occur.

The borate buffered saline solution containing 0.005 weight/volume percent stabilized chlorine dioxide met the before-mentioned criteria for preservatives. However, a control solution of the borate buffered saline solution which did not contain the stabilized chlorine dioxide present did not meet these USP Efficacy criteria for ophthalmics.

### EXAMPLE V

A 21 day subacute eye toxicity study in rabbits was conducted using a borate buffered saline solution containing 0.005 weight/volume percent stabilized chlorine dioxide. The borate buffered saline solution containing the stabilized chlorine dioxide had the following composition:

| Ingredients | Percent (Weight/Volume) |
|---|---|
| Stabilized Chlorine Dioxide | 0.005 |
| Sodium Chloride USP | 0.85 |
| Boric Acid NF | 0.10 |
| Purified Water USP* | To 100 ml |

| | |
|---|---|
| *Quantity sufficient (Q.S.) to provide 100 ml solution. | |

The pH of the above buffered saline solution was adjusted so that the pH of the solution was between 7.7 and 7.9.

The ocular effects of the buffered saline solution containing 0.005 weight/volume percent stabilized chlorine dioxide were evaluated in rabbit eyes in conjunction with Permalens soft contact lenses. Test eye lenses were subjected to daily cleaning, rinsing, and overnight soaking with the borate buffered saline solution containing stabilized chlorine dioxide. Control eye lenses were subjected to the same regimen using preserved normal saline solution. Lenses were fit directly to the eye and worn daily for a minimum of eight hours for 21 consecutive days.

Eyes were observed daily for discomfort at lens insertion and for gross ocular reactions at lens removal. Slit lamp biomicroscopy was performed weekly. Pachometry and rose bengal staining were performed at the conclusion of the experiment. Histopathological evaluation was performed on eyes from three animals. No significant ocular reactions were noted.

The following is a summation of the results of the experiments set forth above:
A. Discomfort: No ocular discomfort was noted at lens insertion throughout the study.
B. Gross Observations: At the time of lens removal, +1 hyperemia was noted in one control eye on Day 17. No other ocular reactions were noted.
C. Slit Lamp Examinations (Days 7, 14 and 21): No ocular reactions were noted in any rabbit.
D. Corneal Metabolism (Days 7, 14 and 21): No test related changes in corneal metabolism, as measured by corneal thickness, were noted throughout the study.
E. Cytotoxicity (Day 21): Rose bengal staining appeared normal in both eyes of all rabbits, indicating that corneal epithelial cell vitality was not affected by the solution tested.
F. Histopathological Evaluation: No microscopic changes which can be specifically related to the test regimen were apparent among the eyes and extraocular tissues examined. There were no predictable microscopic differences observed when comparing the test eyes and extraocular tissues with the control eyes and extraocular tissues.

The above data indicates that a borate buffered saline solution containing 0.005 weight/volume percent stabilized chlorine dioxide, in conjunction with Permalens soft contact lenses, is not discomforting, irritating, toxic or cytotoxic to rabbit eyes following 21 consecutive days of testing.

### EXAMPLE VI

A 1 day acute eye toxicity and cytotoxicity study in rabbits was conducted using a borate buffered saline solution containing 0.005 weight/volume percent stabilized chlorine dioxide. The borate buffered saline solution containing the stabilized chlorine dioxide had the following composition:

| Ingredients | Percent (Weight/Volume) |
|---|---|
| Stabilized Chlorine Dioxide | 0.005 |
| Sodium Chloride USP | 0.85 |
| Boric Acid NF | 0.10 |
| Purified Water USP* | To 100 ml |

| | |
|---|---|
| *Quantity Sufficient (Q.S.) to provide 100 ml solution. | |

The pH of the above buffered saline solution was adjusted so that the pH of the solution was between 7.7 and 7.9.

The ocular effects of the buffered saline solution containing 0.005 weight/volume percent stabilized chlorine dioxide were evaluated in rabbit eyes in conjunction with Permalens soft contact lenses and multiple topical instillations. Test eye lenses were subjected to overnight soaking in the borate buffered saline solution containing 0.005 weight/volume percent stabilized chlorine dioxide followed by direct fit to the eye and 8 hours of wear with topical instillations of the test solution performed at a rate of one drop every one-half hour. Eyes were observed for discomfort and/or gross ocular reactions at lens fit, at each instillation and at lens removal. Slit lamp biomicroscopy was performed following lens removal. Control eyes were subjected to the same regimen using preserved normal saline. No ocular reactions were noted in any rabbit.

The following is a summation of the results of the experiments set forth above:
A. Discomfort: No ocular discomfort was noted at lens fit or at any instillation period throughout the study.
B. Gross Observations: No other ocular reactions were noted at any instillatlon period or at lens removal.
C. Slit Lamp Examinations: No ocular reactions were noted in any rabbit.
D. Cytotoxicity: Rose bengal staining appeared normal in both eyes of all rabbits, indicating that epithelial cell vitality was not affected by the solutions tested.

The above data indicates that a borate buffered saline solution containing 0.005 weight/volume percent stabilized chlorine dioxide, in conjunction with Permalens soft contact lenses, is not discomforting, irritating, toxic or cytotoxic to rabbit eyes following this exaggerated method of testing.

### EXAMPLE VII

An acute eye toxicity and cytotoxicity study in rabbits was conducted using a borate buffered saline solution containing 0.005 weight/volume percent stabilized chlorine dioxide. The borate buffered saline solution containing the stabilized chlorine dioxide had the following composition:

| Ingredients | Percent (Weight/Volume) |
|---|---|
| Stabilized Chlorine Dioxide | 0.005 |
| Sodium Chloride USP | 0.85 |
| Boric Acid NF | 0.10 |
| Purified Water USP* | To 100 ml |

| | |
|---|---|
| *Quantity Sufficient (Q.S.) to provide 100 ml solution. | |

The pH of the above buffered saline solution was adjusted so that the pH of the solution was between 7.7 and 7.9.

The ocular effects of the buffered saline solution containing 0.005 weight/volume percent stabilized chlorine dioxide were evaluated in rabbit eyes following 1 day of multiple topical instillations performed at a rate of one drop every one-half hour for 8 hours. Test eyes were treated with the borate buffered saline solution containing 0.005 weight/volume percent stabilized chlorine dioxide and control eyes were treated with a preserved normal saline solution.

Eyes were observed for discomfort and/or gross ocular reactions at each instillation. Slit lamp biomicroscopy was performed following the last instillatlon period. No ocular reactions were noted in the test eyes.

The following is a summation of the results of the experiments set forth above:
A. Discomfort: +1 discomfort, lasting up to 30 seconds, was noted in the control eye at 3 of 48 instillations involving two of three rabbits.
B. Gross Observations: No ocular reactions were noted at any instillation period.
C. Slit Lamp Examinations: No ocular reactions were noted in any rabbit.
D. Cytotoxicity: Rose bengal staining appeared normal in both eyes of all rabbits, indicating that epithelial cell vitality was not affected by the preparations tested.

The above data indicates that a borate buffered saline solution containing 0.005 weight/volume percent stabilized chlorine dioxide is not discomforting, irritating, toxic or cytotoxic to rabbit eyes following this exaggerated method of testing.

## Claims (Claims for the following Contracting State(s): AT, BE, CH, DE, FR, GB, GR, IT, LI, LU, NL, SE)

1. A preserved ophthalmic formulation comprising an ophthalmically acceptable aqueous medium and, included therein, stabilised chlorine dioxide in an amount in the range of 0.0002 to 0.02 weight/volume percent effective to act as the sole preservative in said formulation, an ophthalmically acceptable buffer component in an amount effective to maintain said formulation at a pH in the range of 6.8 to 8, and an ophthalmically acceptable inorganic salt in an amount effective to maintain the tonicity of said formulation at an ophthalmically acceptable level.

2. The preserved ophthalmic formulation of claim 1 which consists essentially of said ophthalmically acceptable aqueous medium, stabilised chlorine dioxide, said ophthalmically acceptable buffer component and said ophthalmically acceptable inorganic salt.

3. The preserved ophthalmic formulation of claim 1 wherein said stabilised chlorine dioxide is present in an amount in the range of 0.004 to 0.01 weight/volume percent.

4. The preserved ophthalmic formulation of claim 1 wherein the ophthalmically acceptable inorganic salt is selected from the group consisting of alkali metal chlorides, alkaline earth metal chlorides and mixtures thereof.

5. The preserved ophthalmic formulation of claim 1 wherein the ophthalmically acceptable inorganic salt is sodium chloride.

6. The preserved ophthalmic formulation of claim 1 wherein the ophthalmically acceptable inorganic salt is an alkaline earth metal salt selected from the group consisting of calcium chloride, magnesium chloride and mixtures thereof.

7. The preserved ophthalmic formulation of claim 1 wherein the buffer component is selected from the group consisting of potassium phosphates, boric acid, sodium borate, sodium phosphates and mixtures thereof.

8. The preserved ophthalmic formulation of claim 1 which is a solution.

9. The preserved ophthalmic formulation of claim 1 wherein the ophthalmically acceptable inorganic salt is present in an amount in the range of 0.5 to 0.9 weight/volume percent.

10. The preserved ophthalmic formulation of claim 1 wherein the ophthalmically acceptable inorganic salt is sodium chloride and the buffer component is selected from the group consisting of boric acid, sodium borate decahydrate, sodium phosphate dibasic heptahydrate, sodium phosphate monobasic monohydrate and mixtures thereof.

11. A method for preserving an aqueous ophthalmic formulation so as to enhance the shelf life thereof, comprising incorporating into said ophthalmic formulation as the sole preservative, stabilised chlorine dioxide, and a buffer component and an ophthalmically acceptable inorganic salt; the chlorine dioxide, buffer component, inorganic salt and the amounts thereof being as defined in any one of the preceding claims.

## Claims (Claims for the following Contracting State(s): ES)

1. A method for preserving an aqueous ophthalmic formulation so as to enhance the shelf life thereof, comprising incorporating into said ophthalmic formulation as the sole preservative, stabilised chlorine dioxide in an amount in the range from 0.0002 to 0.02 weight/volume percent, an ophthalmically acceptable buffer component in an amount effective to maintain said formulation at a pH in the range of 6.8 to 8, and an ophthalmically acceptable inorganic salt in an amount effective to maintain the tonicity of said formulation at an ophthalmically acceptable level.

2. A method according to claim 1 wherein the preserved ophthalmic formulation consists essentially of the ophthalmically acceptable aqueous medium, stabilized chlorine dioxide, the ophthalmically acceptable buffer component and the ophthalmically acceptable inorganic salt.

3. A method according to claim 1 wherein the stabilised chlorine dioxide is present in an amount in the range from 0.004 to 0.01 weight/volume percent.

4. A method according to claim 1 wherein the ophthalmically acceptable inorganic salt is selected from the group consisting of alkali metal chlorides, alkaline earth metal chlorides and mixtures thereof.

5. A method according to claim 1 wherein the ophthalmically acceptable inorganic salt is sodium chloride.

6. A method according to claim 1 wherein the ophthalmically acceptable inorganic salt is an alkaline earth metal salt selected from the group consisting of calcium chloride, magnesium chloride and mixtures thereof.

7. A method according to claim 1 wherein the buffer component is selected from the group consisting of potassium phosphates, boric acid, sodium borate, sodium phosphates and mixtures thereof.

8. A method according to claim 1 wherein the aqueous ophthalmic formulation is a solution.

9. A method according to claim 1 wherein the ophthalmically acceptable inorganic salt is present in an amount in the range from 0.5 to 0.9 weight/volume percent.

10. A method according to claim 1 wherein the ophthalmically acceptable inorganic salt is sodium chloride and the buffer component is selected from the group consisting of boric acid, sodium borate decahydrate, sodium phosphate dibasic heptahydrate, sodium phosphate monobasic monohydrate and mixtures thereof.

## Patentansprüche (Patentansprüche für folgende(n) Vertragsstaat(en): AT, BE, CH, DE, FR, GB, GR, IT, LI, LU, NL, SE)

1. Konservierte ophtalmische Formulierung, die ein ophtalmisch verträgliches wässriges Medium aufweist, in dem stabilisiertes Chlordioxid mit einem Anteil im Bereich von 0,0002 bis 0,02 Gewichts-/Volumenprozent, das als das einzige Konservierungsmittel in der Formulierung wirksam ist, ein ophtalmisch verträglicher Pufferbestandteil mit einem Anteil, der zum Halten des pH-Wertes der Formulierung in dem Bereich von 6,8 bis 8 wirksam ist, und ein ophtalmisch verträgliches anorganisches Salz mit einem Anteil, der zum Halten der Tonizität der Formulierung bei einem ophtalmisch verträglichen Spiegel wirksam ist, enthalten ist.

2. Konservierte ophtalmische Formulierung nach Anspruch 1, die im wesentlichen aus dem ophtalmisch verträglichen wässrigen Medium, dem stabilisierten Chlordioxid, dem ophtalmisch verträglichen Pufferbestandteil und dem ophtalmisch verträglichen anorganischen Salz besteht.

3. Konservierte ophtalmische Formulierung nach Anspruch 1, bei der das stabilisierte Chlordioxid mit einem Anteil im Bereich von 0,004 bis 0,01 Gewichts-/Volumenprozent vorliegt.

4. Konservierte ophtalmische Formulierung nach Anspruch 1, bei der das ophtalmisch verträgliche anorganische Salz aus der Gruppe ausgewählt ist, die aus Chloriden der Alkalimetalle, Chloriden der Erdalkalimetalle sowie Mischungen davon besteht,

5. Konservierte ophtalmische Formulierung nach Anspruch 1, bei der das opthtalmisch verträgliche anorganische Salz Natriumchlorid ist.

6. Konservierte ophtalmische Formulierung nach Anspruch 1, bei der das ophtalmisch verträgliche anorganische Salz ein Salz der Alkalimetalle ist, das aus der Gruppe ausgewählt ist, die aus Kalziumchlorid, Magnesiumchlorid sowie Mischungen davon besteht.

7. Konservierte ophtalmische Formulierung nach Anspruch 1, bei der der Pufferbestandteil aus der Gruppe ausgewählt ist, die aus Kaluimphosphaten, Borsäure, Natriumborat, Natriumphosphaten und Mischungen davon besteht.

8. Konservierte ophtalmische Formulierung nach Anspruch 1, die eine Lösung ist.

9. Konservierte ophtalmische Formulierung nach Anspruch 1, bei der das ophtalmisch verträgliche anorganische Salz mit einem Anteil im Bereich vom 0,5 bis 0,9 Gewichts-/Volumenprozent vorliegt.

10. Konservierte ophtalmische Formulierung nach Anspruch 1, bei der das ophtalmisch verträgliche anorganische Salz Natriumchlorid ist und der Pufferbestandteil aus der Gruppe ausgewählt ist, die aus Borsäure, Natriumtetraborat-Dekahydrat, Di-Natrium-Phosphat-Heptahydrat, Mono-Natrium-Phosphat-Monohydrat sowie Mischungen davon besteht.

11. Verfahren zur Konservierung einer wässrigen ophtalmischen Formulierung, um deren Gebrauchsfähigkeitsdauer zu verlängern, bei dem in die ophtalmische Fomulierung als das einzige Konservierungsmittel stabilisiertes Chlordioxid und ein Pufferbestandteil und ein ophtalmisch verträgliches anorganisches Salz eingebracht werden, wobei das Chlordioxid, der Pufferbestandteil, das anorganische Salz sowie deren Anteile durch einen der vorhergehenden Ansprüche definiert sind.

## Patentansprüche (Patentansprüche für folgende(n) Vertragsstaat(en): ES)

1. Verfahren zur Konservierung einer wässrigen ophtalmischen Formulierung, um deren Gebrauchsfähigkeitsdauer zu verlängern, bei dem in die ophtalmische Formulierung als das einzige Konservierungsmittel stabilisiertes Chlordioxid mit einem Anteil im Bereich von 0,0002 bis 0,02 Gewichts-/Volumenprozent, ein ophtalmisch verträglicher Pufferbestandteil mit einem Anteil, der zum Halten des pH-Wertes der Formulierung in dem Bereich von 6,8 bis 8 wirksam ist, und ein ophtalmisch verträgliches anorganisches Salz mit einem Anteil, der zum Halten der Tonizität der Formulierung bei einem ophtalmisch verträglichen Spiegel wirksam ist, eingebracht werden.

2. Verfahren nach Anspruch 1, bei dem die konservierte ophtalmische Formulierung im wesentlichen aus dem ophtalmisch verträglichen wässrigen Medium, stabilisiertem Chlordioxid, dem ophtalmisch verträglichen Pufferbestandteil und dem ophtalmisch verträglichen anorganischen Salz besteht.

3. Verfahren nach Anspruch 1, bei dem das stabilisierte Chlordioxid mit einem Anteil im Bereich von 0,004 bis 0,01 Gewichts-/Volumenprozent vorliegt.

4. Verfahren nach Anspruch 1, bei das ophtalmisch verträgliche anorganische Salz aus der Gruppe ausgewählt ist, die aus Chloriden der Alkalimetalle, Chloriden der Erdalkalimetalle sowie Mischungen davon besteht.

5. Verfahren nach Anspruch 1, bei dem das ophtalmisch verträgliche anorganische Salz Natriumchlorid ist.

6. Verfahren nach Anspruch 1, bei dem das ophtalmisch verträgliche anorganische Salz ein Salz der Erdalkalimetalle ist, das aus der Gruppe ausgewählt ist, die aus Kalziumchlorid, Magnesiumchlorid sowie Mischungen davon besteht.

7. Verfahren nach Anspruch 1, bei dem der Pufferbestandteil aus der Gruppe ausgewählt ist, die aus Kaliumphossphaten, Borsäure, Natriumborat, Natriumphosphaten sowie Mischungen davon besteht.

8. Verfahren nach Anspruch 1, bei dem die wässrige ophtalmische Formulierung eine Lösung ist.

9. Verfahren nach Anspruch 1, bei dem das ophtalmisch verträgliche anorganische Salz mit einem Anteil im Bereich von 0,5 bis 0,9 Gewichts-/Volumenprozent vorliegt.

10. Verfahren nach Anspruch 1, bei dem das ophtalmisch verträgliche anorganische Salz Natriumchlorid ist und der Pufferbestandteil aus der Gruppe ausgewählt ist, die aus Borsäure, Natriumtetraborat-Dekahydrat, Di-Natrium-Phosphat-Heptahydrat, Mono-Natrium-Phosphat-Monohydrat sowie Mischungen davon besteht.

## Revendications (Revendications pour l'(les) Etat(s) contractant(s) suivant(s): AT, BE, CH, DE, FR, GB, GR, IT, LI, LU, NL, SE)

1. Formulation ophtalmique de conservation comprenant un milieu aqueux ophtalmiquement acceptable et dans lequel est inclus du dioxyde de chlore stabilisé en une quantité comprise entre 0,0002 et 0,02 % poids/volume efficace pour servir de conservateur unique dans ladite formulation, un tampon ophtalmiquement acceptable en une quantité efficace pour maintenir ladite formulation à un pH de 6,8 à 8, et un sel inorganique ophtalmiquement acceptable en une quantité efficace pour maintenir la tonicité de ladite formulation à un niveau ophtalmiquement acceptable.

2. Formulation ophtalmique de conservation selon la revendication 1, qui est composée essentiellement dudit milieu aqueux ophtalmiquement acceptable, de dioxyde de chlore stabilisé, dudit tampon ophtalmiquement acceptable et dudit sel inorganique ophtalmiquement acceptable.

3. Formulation ophtalmique de conservation selon la revendication 1, dans laquelle ledit dioxyde de chlore stabilisé est présent en une quantité comprise entre 0,004 et 0,01 % poids/volume.

4. Formulation ophtalmique de conservation selon la revendication 1, dans laquelle le sel inorganique ophtalmiquement acceptable est choisi dans l'ensemble formé par les chlorures de métal alcalin, les chlorures de métal alcalino-terreux et des mélanges de ceux-ci.

5. Formulation ophtalmique de conservation selon la revendication 1, dans laquelle le sel inorganique ophtalmiquement acceptable est le chlorure de sodium.

6. Formulation ophtalmique de conservation selon la revendication 1, dans laquelle le sel inorganique ophtalmiquement acceptable est un sel de métal alcalino-terreux choisi dans l'ensemble formé par le chlorure de calcium, le chlorure de magnésium et des mélanges de ceux-ci.

7. Formulation ophtalmique de conservation selon la revendication 1, dans laquelle le tampon est choisi dans l'ensemble formé par les phosphates de potassium, l'acide borique, le borate de sodium, les phosphates de sodium et des mélanges de ceux-ci.

8. Formulation ophtalmique de conservation selon la revendication 1 qui se présente sous la forme d'une solution.

9. Formulation ophtalmique de conservation selon la revendication 1, dans laquelle le sel inorganique ophtalmiquement acceptable est présent en une quantité comprise entre 0,5 et 0,9 % poids/volume.

10. Formulation ophtalmique de conservation selon la revendication 1, dans laquelle le sel inorganique ophtalmiquement acceptable est le chlorure de sodium et le tampon est choisi dans l'ensemble formé par l'acide borique, le borate de sodium décahydraté, le phosphate de sodium dibasique heptahydraté, le phosphate de sodium monobasique monohydraté et des mélanges de ceux-ci.

11. Procédé de conservation d'une formulation aqueuse ophtalmique, de manière à en améliorer la durée de conservation, comprenant l'incorporation dans ladite formulation ophtalmique, à titre d'unique conservateur, du dioxyde de chlore stabilisé, ainsi qu'un tampon et un sel inorganique ophtalmiquement acceptable ; le dioxyde de chlore, le tampon et le sel inorganique étant présents en des quantités telles que définies dans l'une quelconque des revendications précédentes.

## Revendications (Revendications pour l'(les) Etat(s) contractant(s) suivant(s): ES)

1. Procédé de conservation d'une formulation aqueuse ophtalmique, de manière à en améliorer la durée de conservation, comprenant l'incorporation dans ladite formulation ophtalmique, à titre d'unique conservateur, du dioxyde de chlore stabilisé en une quantité comprise entre 0,0002 et 0,02 % poids/volume, un tampon ophtalmiquement acceptable en une quantité efficace pour maintenir ladite formulation à un pH de 6,8 à 8, et un sel inorganique ophtalmiquement acceptable en une quantité efficace pour maintenir la tonicité de ladite formulation à un niveau ophtalmiquement acceptable.

2. Procédé selon la revendication 1, dans lequel la formulation ophtalmique est composée essentiellement du milieu aqueux ophtalmiquement acceptable, de dioxyde de chlore stabilisé, du tampon ophtalmiquement acceptable et du sel inorganique ophtalmiquement acceptable.

3. Procédé selon la revendication 1, dans lequel le dioxyde de chlore stabilisé est présent en une quantité comprise entre 0,004 et 0,01 % poids/volume.

4. Procédé selon la revendication 1, dans lequel le sel inorganique ophtalmiquement acceptable est choisi dans l'ensemble formé par les chlorures de métal alcalin, les chlorures de métal alcalino-terreux et des mélanges de ceux-ci.

5. Procédé selon la revendication 1, dans lequel le sel inorganique ophtalmiquement acceptable est le chlorure de sodium.

6. Procédé selon la revendication 1, dans lequel le sel inorganique ophtalmiquement acceptable est un sel de métal alcalino-terreux choisi dans l'ensemble formé par le chlorure de calcium, le chlorure de magnésium et des mélanges de ceux-ci.

7. Procédé selon la revendication 1, dans lequel le tampon est choisi dans l'ensemble formé par les phosphates de potassium, l'acide borique, le borate de sodium, les phosphates de sodium et des mélanges de ceux-ci.

8. Procédé selon la revendication 1, dans lequel la formulation ophtalmique aqueuse est une solution.

9. Procédé selon la revendication 1, dans lequel le sel inorganique ophtalmiquement acceptable est présent en une quantité comprise entre 0,5 et 0,9 % poids/volume.

10. Procédé selon la revendication 1, dans lequel le sel inorganique ophtalmiquement acceptable est le chlorure de sodium et le tampon est choisi dans l'ensemble formé par l'acide borique, le borate de sodium décahydraté, le phosphate de sodium dibasique heptahydraté, le phosphate de sodium monobasique monohydraté et des mélanges de ceux-ci.
